(19) Europäisches Patentamt
European Patent Office
Office européen des brevets

(11) Publication number: 0 335 745 A1

# (12) EUROPEAN PATENT APPLICATION

(21) Application number: 89303228.4

(22) Date of filing: 31.03.89

(51) Int. Cl.4: C 07 D 213/69
A 61 K 31/44

(30) Priority: 31.03.88 US 176061

(43) Date of publication of application:
04.10.89 Bulletin 89/40

(84) Designated Contracting States:
AT BE CH DE ES FR GB GR IT LI LU NL SE

(71) Applicant: THE ROYAL FREE HOSPITAL SCHOOL OF MEDICINE
Rowland Hill Street
London NW3 2PF (GB)

(72) Inventor: Kontoghiorghes, George John
5 Seelig Avenue West Hendon
GB-London NW9 7BB (GB)

Sheppard, Lyndon Norman
19 Alton Cottages Eynsford
GB-Kent DA4 OAX (GB)

(74) Representative: Cresswell, Thomas Anthony et al
J.A. Kemp & Co. 14 South Square Gray's Inn
London WC1R 5EU (GB)

## (54) Process for producing pyrid-4-ones.

(57) Substituted-3-hydroxy-pyrid-4-ones, derivatives thereof, and salts thereof are produced by reacting subsituted-3-hydroxypyr-4-one with ammonia or a non-sterically hindered primary amine usually in the presence of a solvent such as water. Also disclosed are novel derivatives of 2-ethyl-3-hydroxypyrid-4-one.

EP 0 335 745 A1

**Description**

## PROCESS FOR PRODUCING PYRID-4-ONES

FIELD OF THE INVENTION

The present invention relates to a process for producing substituted-3-hydroxypyrid-4-one and derivatives thereof which are useful as chelating agents in, for instance, the treatment of diseases involving iron overload. The invention also relates to certain novel 2-alkyl-3-hydroxypyrid-4-ones which may be produced by this process.

BACKGROUND OF THE INVENTION

Various hydroxypyrid-2- and 4-ones have previously been disclosed as iron chelating agents in, for instance, British Patent Specification No. 2,118,176. Synthetic methods for the production of these compounds have been described in this Patent and in T. Severin and A. Loidl, Z. Lebensm, Unters-Forsch., 161, 119 (1976), M. Yasue, N. Kawamura and J. Sakakibara, Yakugaku Zasshi, 90, 1222 (1970), and G.J. Kontoghiorghes, Ph.D. Thesis, Essex University, British Library Microfilm D66194/86, 1982 but these all suffer in that they are multi-step processes. Typical of these processes is that illustrated in Scheme 1 below:

SCHEME 1

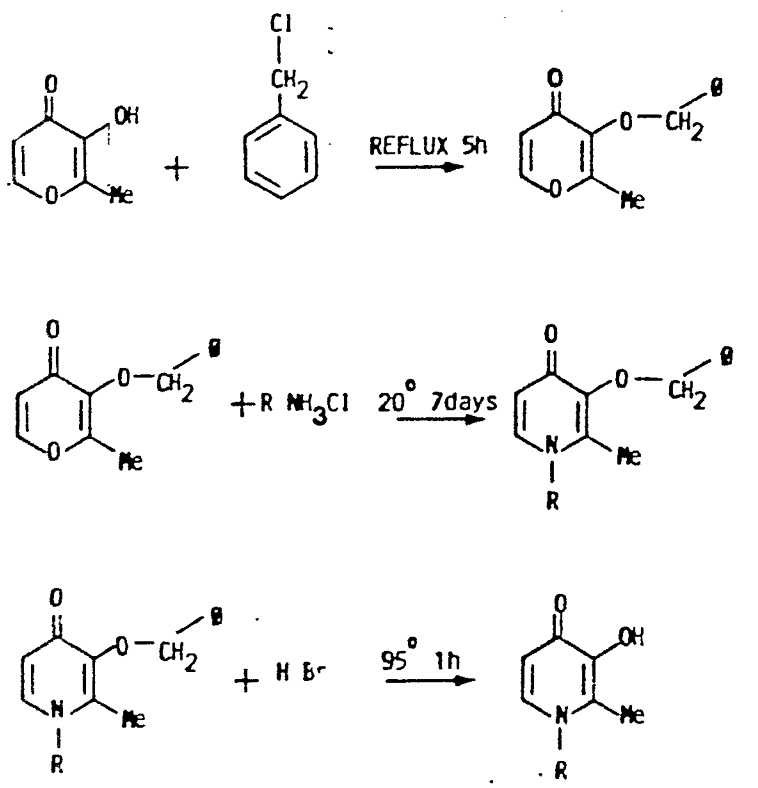


R=Me, Et, nPr.

and such a process is described in, for instance, British Patent 2,118,176. In addition to being a multi-step process, this process is particularly disadvantageous because of the use of benzyl chloride; this highly toxic compound must carefully be removed from the final product if the product is to be used in medicine.

SUMMARY OF THE INVENTION

Accordingly, it is an object of the present invention to provide a simple process for producing substituted-3-hydroxypyrid-4-ones.

The invention therefore provides a process for producing substituted-3-hydroxypyrid-4-ones and derivatives thereof and salts thereof, which process comprises reacting a substituted-3-hydroxypyr-4-one with ammonia or a non-sterically hindered primary amine or an acid addition salt thereof.

## BRIEF DESCRIPTION OF THE DRAWINGS

Fig 1: pH titration of the chelator iron complexes. Chelators (2mM in 0.1M NaCl, 25ml) and freshly prepared iron (III) chloride (0.5mM in 0.1M NaCl, 25ml) were mixed and titrated with HCl (10M) and NaOH (5M). The absorbance of the chelator iron mixture at 460nm was measured after each pH change. 1-Methyl- (Δ), 1-ethyl- (□) and 1-propyl- (◊) -2-ethyl-3-hydroxypyrid-4-ones.

Fig 2:

A. Job plots of the 1-alkyl-2-ethyl-3-hydroxypyrid-4-ones with iron at pH 7.3. Chelators (2mM in 0.1mM NaCl 0-5ml) and iron (2mM in 0.1M NaCl, 0-5ml) were mixed at different molar ratios to a final volume of 5ml. 4 x PBS (5ml), pH 7.3 was then added and the absorbance at 460nm measured. 1-Methyl- (Δ), 2-ethyl- (□), 1-propyl (◊) 2-ethyl-3-hydroxypyrid-4-ones.

B. Theoretical plots of 1:1,2:1 and 3:1 chelator:iron complexes. Theoretical Job plots for 1:1, 2:1, 3:1 chelator:iron complexes were obtained using the maximum absorbance values from the Job plots in A. 1:1 (◊) 2:1 (□), 3:1 (Δ).

Fig. 3: Iron mobilisation from human ferritin and haemosiderin using 1-alkyl-2-ethyl-3-hydroxypyrid-4-ones. Ferritin (0.25mg iron 10.7% w/w iron, 1.5ml) and haemosiderin (0.25mg iron, 16.5% w/w iron, 1.5ml) were dialysed against a chelator solution (4x10$^{-3}$M, 10ml) in 2 x PBS, pH 7.3, 37° C. The progress of the reaction was followed by measuring the absorbance at 460nm of the dialysate at different time intervals. ELINMe; 2-ethyl-3-hydroxy-1-methylpyrid-4-one; ELINEt; 1,2-diethyl-3-hydroxypyrid-4-one; ELINPr: 2-ethyl-3-hydroxy-1-propylpyrid-4-one; ferritin ▲ ■), haemosiderin (Δ□).

## DETAILED DESCRIPTION OF THE INVENTION

In accordance with the invention, a substituted-3-hydroxypyrid-4-one or a salt thereof or a derivative of substituted-3-hydroxypyrid-4-one or a salt of said derivative is produced from the correspondingly substituted-3-hydroxypyr-4-one. The substituent is usually at the 2-position and is a group which does not sterically hinder reaction at the 1-oxygen position of the pyrone and is preferably an alkyl, such as a methyl or ethyl, group. The process of the invention may also be used in the production of pyrid-4-ones bearing substituents in the 5-and/or 6-positions, again subject to the need to avoid steric hinderance at the 1-position. The salts of the subsituted-3-hydroxypyrid-4-one and derivatives which may be produced in accordance with the present invention include acid addition salts of mineral and organic acids, particularly the hydrohalic acids and especially the hydrochloride salts.

When a substituted-3-hydroxypyr-4-one is treated with ammonia, the resultant compound is the corresponding substituted-3-hydroxypyrid-4-one, whereas reaction with the corresponding non-sterically hindered primary amine results in the corresponding N-substituted-3-hydroxypyrid-4-one. When forming the N-substituted derivatives it is important that the primary amino group is accessible for reaction with the substituted-3-hydroxypyr-4-one and any bulky group in the amine should, therefore, be separated from the primary amino group by at least one, for instance one or two or more, methylene groups. One sub-class of amines which may be used in the present process are the alkylamines, for instance lower n-alkylamines such as methylamine, ethylamine and n-propylamine. Subject to the requirement to avoid bulky groups adjacent the primary amino group, such amines may optionally contain branched and/or substituted alkyl groups. Suitable substituents include hydroxy, carboxy, alkenyl, alkoxy, acyl, acyloxy, amino and mono- and di-alkylamino substitutent. It is also possible to use amine reagents having more than one, for instance two or three primary amino groups and, in general, the substituted-3-hydroxypyr-4-one starting material will react with any non-sterically hindered primary amino groups on such a reagent.

The process of the present invention may be conducted under any suitable conditions and it is contemplated that this will include reaction of the substituted-3-hydroxypyr-4-one with ammonia or a non-sterically hindered primary amine in the absence of a solvent, particularly when the amine is a liquid. Special measures may be required in order to react a substituted-3-hydroxypyr-4-one with ammonia or a gaseous amine such as methylamine or ethylamine as will be apparent to those skilled in the art. It is, however, preferred to conduct the process in the presence of a suitable solvent which may be, for instance, water or an inert organic solvent such as a lower alkanol, for instance methanol or ethanol, or in the presence of a mixture of solvents, such as aqueous methanol. When the reaction is conducted in the presence of a solvent, it is generally conducted at elevated temperature, for instance at the reflux temperature of the reaction mixture, usually under atmospheric pressure.

Preferably the amine reagent is used in an amount of from 1 to 5 more preferably from 1.5 to 3 equivalents based on the amount of pyrone starting material.

The ammonia or primary amine may be used in the form of the free base or in the form of an acid addition salt, for instance the addition salt of a mineral or organic acid, preferably a hydrohalic acid, and most preferably as a hydrochloride salt. When the ammonia or primary amine is used in the form of an acid, addition salt the substituted-3-hydroxypyrid-4-ones will be produced in the form of the corresponding salt. If it is desired to produce the free base, the acid may be neutralised using a suitable base such as an alkali, especially sodium hydroxide, which may be incorporated in the reaction mixture together with the substituted-3-hydroxypyr-4-one and the ammonium or amine salt. Alternatively, reaction of a salt of ammonia or an amine with the

substituted-3-hydroxypyr-4-one results in formation of the substituted-3-hydroxy-pyrid-4-one salt which is then converted to the free base by treatment with a base such as an alkali, for instance sodium hydroxide. Such conversion may be conducted in situ or after isolation or purification of the desired substituted-3-hydroxypyrid-4-one.

It will usually be necessary in order to obtain a pure product to isolate and purify the substituted-3-hydroxypyrid-4-one produced according to the process of this invention. Such isolation and purification can be effected by conventional techniques such as recrystallisation from suitable solvents, for instance water. The purification and isolation may also include steps of converting a substituted-3-hydroxypyrid-4-one salt to the free base and/or converting a free substituted-3-hydroxypyrid-4-one base to a salt thereof by methods generally described above.

The process of the present invention may be used for producing a wide variety of substituted-3-hydroxypyrid-4-one derivatives, and salts thereof, many of which are already known in the literature. The process may also be used for producing certain novel compounds, including the following:

1,2-diethyl-3-hydroxypyrid-4-one,
2-ethyl-3-hydroxy-1-propylpyrid-4-one,
2-ethyl-3-hydroxypryid-4-one,
3-hydroxy-1-(2-methoxyethyl)-2-methylpyrid-4-one,
2-ethyl-3-hydroxy-1-(2-hydroxypropyl)pyrid-4-one,
2-ethyl-3-hydroxy-1-(2-hydroxyethyl)-pyrid-4-one,
2-ethyl-3-hydroxy-1-(2-methoxyethyl)-pyrid-4-one,
3-hydroxy-2-methylpyrid-4-one,
1-allyl-3-hydroxy-2-methylpyrid-4-one,
3-hydroxy-1-(2-hydroxypropyl)-2-methylpyrid-4-one,
1-(2,3-dihydroxypropyl)-3-hydroxy-2-methylpyrid-4-one,
1-allyl-2-ethyl-3-hydroxypyrid-4-one,
1-(2,2-diethoxyethyl)-3-hydroxy-2-methylpyrid-4-one,
1-2-diethylaminoethyl-3-hydroxy-2-methylpyrid-4-one,
1-(3-ethoxypropyl)-3-hydroxy-2-methylpyrid-4-one,
1-(3-ethoxypropyl)-2-ethyl-3-hydroxypyrid-4-one.

In a further aspect the invention also provides pharmaceutical formulations comprising any one of the above novel compounds or a salt thereof, in a pharmaceutically effective amount, together with a pharmaceutically acceptable carrier or diluent therefor.

The invention further provides a method for treating the human or animal body comprising administering any one of the foregoing novel compounds or an acid addition salt thereof in an effective non-toxic amount to a human or animal in need thereof. Suitable formulations and methods for treating humans and animals using any one of the above novel compounds or an acid addition salt thereof are substantially as described in the literature in connection with 1-alkyl-2-methyl-3-hydroxypyrid-4-one such as is described in the British Medical Journal, 295, 1509 (1987) and the Lancet i; 1294(1987).

Variations and modifications of the process according to the present invention will be apparent to those skilled in the art. All such modifications and variations are considered within the scope of the invention.

The following Examples illustrate certain aspects of the present invention that are not intended to limit the scope of the invention as defined in the claims.

EXAMPLE 1

1,2-Dimethyl-3-hydroxypyrid-4-one was prepared from the reflux for 6.5 h of 3-hydroxy-2-methylpyr-4-one (maltol) (10g) with 3 equivalents of aqueous methylamine (40%) in 200ml of water. Decolourising charcoal was added after refluxing and the mixture left for 0.5 h. This was then filtered and the dark brown filtrate was evaporated in vacuo to give a dark brown solid. Crystallisation from hot water gave fine white needles of 1,2-dimethyl-3-hydroxypyrid-4-one (50%) yield. $^{1}H$ NMR (250 MHz; $D_2O$); $\delta$ 2.24 (3 H, s, 2-$CH_3$), 3.61 3 H, s, 1-$CH_3$), 6.33 and 7.46 (2 H, ABq J 7 Hz, H-5 and H-6).

EXAMPLE 2

1-Ethyl-3-hydroxy-2-methylpyrid-4-one was prepared in a similar manner to 1,2-dimethyl-3-hydroxypyrid-4-one using 3 equivalents of aqueous ethylamine (70%). Crystallisation from hot methanol gave brown crystals which on recrystallisation from the same solvent gave 1-ethyl-3-hydroxy-2-methylpyrid-4-one as white octahedra (24% yield), $^{1}H$ NMR (250 MHz; $D_2O$): $\delta$ 1.20 (3 H, t J 7 Hz, -$CH_2CH_3$), 2.27 (3 H,s, 2-$CH_3$), 3.94 (2 H, q J 7 Hz,-$CH_2CH_3$), 6.36 and 7.50 (2 H, ABq J 7 Hz, H-5 and H-6).

EXAMPLE 3

3-Hydroxy-2-methyl-1-propylpyrid-4-one was prepared in a similar manner to that of Example 1 using 3 equivalents of neat n-propylamine and 200ml water. Crystallistion from hot acetone gave an orange brown solid which, on recrystallisation yielded colourless blades (21% yield), $^{1}H$ nmr (250 MHz;$D_2O$):$\delta$ 0.72 (3H,t,J7Hz, -$CH_2CH_2CH_3$), 1.56 (2H,t,J7Hz, -$CH_2CH_2CH_3$), 2.22 (3H,s,2-$CH_3$), 3.82 (2H, dq J7Hz, $CH_2CH_2CH_3$), 6.32 (1H,d J7Hz, H-5) and 7.45 (1H, d J7Hz, H-6).

EXAMPLE 4

2-Ethyl-3-hydroxy-1-methylpyrid-4-one was prepared from ethyl maltol (Pfizer, UK) (30.0g), 40% aqueous methylamine (55.4ml, 3 equiv.) and water (600ml) by refluxing for 7 h. The mixture was then allowed to cool, rotary evaporated in vacuo forming a thick black paste which on three recrystallisations from methanol gave white crystals m.pt 199-202°C (with decomposition).

EXAMPLES 5 to 20

By methods analogous to those of Examples 1 to 4 but using appropriate amine reagents, the compounds of Examples 5 to 20 were produced. In most cases repeated recrystallisations were required to obtain pure, white crystals.

| Example No. | Compound | mpt (°C) |
|---|---|---|
| 5 | 1,2-diethyl-3-hydroxy-pyrid-4-one | 179-182 (with decomp) |
| 6 | 2-ethyl-3-hydroxy-1-propylpyrid-4-one | 129-132 (no decomp) |
| 7 | 2-ethyl-3-hydroxypyid-4-one | 225-228 (decomp) |
| 8 | 3-hydroxy-1-(2-methoxyethyl)-2-methylpyrid-4-one | 185-187.5 (no decomp) |
| 9 | 2-ethyl-3-hydroxy-1-(2-hydroxypropyl)pyrid-4-one | 203-207 (no decomp) |
| 10 | 2-ethyl-3-hydroxy-1-(2-hydroxyethyl)-pyrid-4-one | 175-177 (no decomp) |
| 11 | 2-ethyl-3-hydroxy-1-(2-methoxyethyl)-pyrid-4-one | 154-158 (no decomp) |
| 12 | 3-hydroxy-2-methyl pyrid-4-one | Blackened over range 280-286; no m.p. as such |
| 13 | 1-allyl-3-hydroxy-2-methylpyrid-4-one | 157-159 (no decomp) |
| 14 | 3-hydroxy-1-(2-hydroxypropyl)-2-methylpyrid-4-one | 178-180 (no decomp) |
| 15 | 1-(2,3-dihydroxypropyl)-3-hydroxy-2-methylpyrid-4-one | 173-174.5 (no decomp) |
| 16 | 1-allyl-2-ethyl-3-hydroxypyrid-4-one | 152-155 (no decomp) |
| 17 | 1-(2,2-diethoxyethyl)-3-hydroxy-2-methylpyrid-4-one | 115-117 (no decomp) |

| Example No. | Compound | mpt ($^{o}C$) |
| --- | --- | --- |
| 18 | 1-(2-diethylaminoethyl)-3-hydroxy-2-methylpyrid-4-one | 144.5-146.5 (no decomp) |
| 19 | 1-(3-ethoxypropyl)-3-hydroxy-2-methylpyrid-4-one | 90.5-92.5 (no decomp) |
| 20 | 1-(3-ethoxypropyl)-2-ethyl-3-hydroxypyrid-4-one | 84.5-86.5 (no decomp) |

The compounds of Examples 4 to 20 have not previously been described. Further characterising data is presented in Tables 1 to 4 below. Yields obtained in the above Examples are given in Table 5 below.

TABLE 1

250 MHz $^{1}H$ NMR Data for 1-alkyl-2-ethyl-hydroxypyrid-4-ones recorded in $D_2O$ in p.p.m*

| Compound of Example No. | 1-Alkyl | 2-substituent | H-5 | H-6 |
|---|---|---|---|---|
| 4 | 3.68 (3H,s N-$CH_3$) | 1.05 (3H,tJ 8 Hz, -$CH_3$) 2.71 (2H,qJ 8 Hz,-$CH_2$) | 6.35 (dJ7Hz) | 7.46 (dJ7Hz) |
| 5 | 1.25 (3H,tJ 7Hz -$CH_2CH_3$) 3.98 (2H,qJ 7 Hz,-$CH_2$CH3) | 1.06 (3H,tJ 8 Hz,-$CH_3$) 2.71 (2H,qJ 8 Hz,-$CH_2$) | 6.39 (dJ7Hz) | 7.52 (dJ7Hz) |
| 6 | 0.78 (3H,tJ7Hz -$CH_2CH_2CH_3$) 1.64 (2H,tqJ 7Hz,-$CH_2CH_2$ $CH_3$) 3.90 (2H, tJ8Hz -$CH_2CH_2CH_3$) | 1.06 (3H,tJ 8 Hz,-$CH_3$) 2.71 (2H,qJ 7 Hz,-$CH_2$) | 6.38 (dJ7Hz) | 7.51 (dJ7Hz) |
| 7 | N/A | 1.06 (3H,tJ 8Hz, -$CH_3$), 2.59 (2H,qJ 8Hz, -$CH_2$-) | 6.36 (dJ7Hz) | 7.43 (dJ7Hz) |
| 8 | 3.16(3Hs-$OCH_3$) 3.62 (2HtJ5Hz, >N-$CH_2$-), 4.12 (2H,t,J5Hz, -$CH_2$O-) | 2.27 (3H,s-$CH_3$) | 6.35 (dJ7Hz) | 7.48 (dJ7Hz) |

| Compound of Example No. | 1-Alkyl | 2-substituent | H-5 | H-6 |
|---|---|---|---|---|
| 9 | 1.10 (3H,d*J*6Hz, $-CH_3$), 3.74-4.12 (3H,m, >N-C*H*$_2$-C*H*(OD)-) | 1.02 (3H,t*J*8Hz $-CH_3$), 2.58-2.85 (2H,m -C*H*$_2$-) | 6.36 (d*J*7Hz) | 7.48 (d*J*7Hz) |
| 10 | 3.74 (2H,t*J*5Hz $-CH_2OD$), 4.09 (2H,t*J*5Hz, >N-C*H*$_2$-). | 1.03 (3H,t*J*8Hz, $-CH_3$), 2.72 (2H,q*J*8Hz -C*H*$_2$-) | 6.37 (d*J*7Hz) | 7.50 (d*J*7Hz) |
| 11 | 3.18 (3H,s- $OCH_3$), 3.65 (2H,t*J*5Hz >N-C*H*$_2$-), 4.16 (2H,t*J*5Hz, -C*H*$_2$-0-) | 1.04 (3H,tJ8Hz, $-CH_3$) 2.72 (2H,q*J*8Hz, -C*H*$_2$-) | 6.37 (d*J*7Hz) | 7.50 (d*J*7Hz) |
| 12+ | 4.45 (br s, >N-*H*) | 2.17 (3H,s,$-CH_3$) | 6.09 (d*J*7Hz) | 7.40 (d*J*7Hz) |
| 13++ | 4.58-4.61 (2H,m,-C*H*$_2$-CH=$CH_2$),4.88 [1H, dd (A of ~ABX) *J* 1 and 17Hz, *E*-$CH_2$-CH=CH-*H*], 5.21 [1H,~dd (B of ~ABX) *J* 1 and 10Hz, *Z*-$CH_2$-CH=CH-*H*], 5.92-6.07 (1*H*,m $-CH_2$-C*H*=$CH_2$) | 2.24 (3H,s-C*H*$_3$) N.B. Substituent -3: 4.32 (br s, -O*H*) | 6.14 (d*J*7Hz) | 7.54 (d*J*7Hz) |
| 14 | 1.11 (3H,d*J*6Hz $-CH_3$), 3.74-4.09 (3H,m, >N-$CH_2$-CH<) | 2.28 (3H,s-$CH_3$) | 6.36 (d*J*7Hz) | 7.48 (d*J*7Hz) |

| Compound of Example No. | 1-Alkyl | 2-substituent | H-5 | H-6 |
|---|---|---|---|---|
| 15 | 3.46-3.59 (2H, m, $-CH_2$), 3.82-3.92 (2H, m, $-CH_2-$), 4.14-4.25 [(1H, m, >CH(OD)] | 2.31 (3H, s-$CH_3$) | 6.38 (dJ7Hz) | 7.51 (dJ7Hz) |
| 16 | 4.52-4.64 (2H, m, $-CH_2-CH=CH_2$) 4.77 (1H, d J 17Hz, E-$CH_2$-CH=CH-H), 5.14 (1H, dJ11Hz, Z-$CH_2$-CH=CH-H) 5.84-5.98 (1H, m $-CH_2-CH=CH_2$) | 1.05 (3H, tJ8Hz $-CH_3$), 2.66 (2H, qJ8Hz $-CH_2-$) | 6.41 (dJ7Hz) | 7.48 (dJ7Hz) |
| 17+++ | 0.96 (6H, tJ7Hz, 2 x $-CH_3$), 3.34-3.46 (2H, m, $-O-CH_2-$), 3.57-3.69 (2H, m, $-O-CH_2-$), 4.06 (2H, dJ5Hz >N-$CH_2$-) 4.72 (1H, tJ5Hz, $-CH_2-CH$<) | 2.31 (3H, s-$CH_3$) | 6.37 (dJ7Hz) | 7.50 (dJ7Hz) |
| 18 | 0.91 (6H, tJ7Hz, 2 x $-CH_2CH_3$), 2.51 (4H, qJ7Hz, 2 x $CH_2CH_3$), 2.69-2.76 [2H m, $-CH_2(CH_2CH_3)_2$], 4.04-4.10 (2H, m, ring-$CH_2$). | 2.30 (3H, s, ring $-CH_3$) | 6.37 (dJ7Hz) | 7.51 (dJ7Hz) |

| Compound of Example No. | 1-Alkyl | 2-substituent | H-5 | H-6 |
|---|---|---|---|---|
| 19 | 0.97 (3H, t$\underline{J}$7Hz, $-CH_3$, 1.82-1.92 (2H, m, $-CH_2CH_2CH_2-$), 3.29-3.37 (4H, m, $-CH_2-$ and $-OCH_2CH_3$), 4.02 (2H, t$\underline{J}$7Hz, $-CH_2-$) | 2.27 (3H, s, $-CH_3$) | 6.35 (d$\underline{J}$7Hz) | 7.49 (d$\underline{J}$7Hz) |
| 20 | 0.98 (3H, t$\underline{J}$Hz $-CH_3$, 1.82-1.94 (2H, m, $-CH_2CH_2CH_2-$), 3.30-3.38 (4H, m, $-CH_2-$ and $-OCH_2CH_3$), 4.04 (2H, t$\underline{J}$7Hz, $-CH_2-$) | 1.04 (3H, t, $\underline{J}$8Hz, $-CH_3$), 2.71 (2H, q$\underline{J}$7Hz, $-CH_2-$) | 6.36 (d$\underline{J}$7Hz) | 7.49 (d$\underline{J}$7Hz) |

* Multiplicities s,d,t and q denote singlet, doublet, triplet and quartet respectively, reported from HOD at 4.65 p.p.m.

\+ In DMSO -d6 with TMS
++ In DMSO -d6 with TMS
+++ In $D_2O$ with TMS (HOD at 4.67 ppm)

TABLE 2

Infra Red Data

| Compound of Example No. | Vmax (KBr)$cm^{-1}$ |
|---|---|
| 4 | 3480 (O-H stretch), 1627 (C=O stretch) |
| 5 | 3480 (O-H stretch), 1621 (C=O stretch) |
| 6 | 3480 (O-H stretch), 1626 (C=O stretch) |
| 7 | 3530m sh, 1647s, 1626s |
| 8 | 3840m (O-H stretching), 1628s (C=O stretching) |
| 9 | 3440m br sh (O-H stretching), 1625s (C=O stretching) |
| 10 | ca 3500m br (O-H stretching), 1630s (C=O stretching) |
| 11 | 3480m (O-H stretching), 1620s (C=O stretching) |
| 12 | 3410m br sh (O-H and N-H stretching), 1647s, 1619s |
| 13 | 3360m (O-H stretching), 1629s (C=O stretching) |
| 14 | 3440m (O-H stretching), 1630s (C=O stretching) |
| 15 | 3430m sh (O-H stretching), 1626s (C=O stretching) |
| 16 | 3470m (O-H stretching), 1622s (C=O stretching) |
| 17 | 3420m (O-H stretching), 1626s (C=O stretching) |
| 18 | 3470m (O-H stretching), 1630s (C=O stretching) |
| 19 | 3380m (O-H stretching), 1629s (C=O stretching) |
| 20 | 3500m (O-H stretching), 1622s (C=O stretching) |

TABLE 3

| Compound of Example No. | M+(% Rel. abundance) | Found | Required |
|---|---|---|---|
| 4 | 153(100) | M+ 153.0781 $C_8N_{11}NO_2$ | 153.0790 |
| 5 | 167(68) | $M^+$ 167.0953 $C_9H_{13}NO_3$ | 167.0946 |
| 6 | 181(35) | $M^+$ 181.1103 $C_{10}H_{15}NO_2$ | 181.1103 |
| 7 | 139(88) | $M^+$-1 138.0562 $C_7H_9NO_2$ | 138.0555 |
| 8 | 183(100) | $M^+$ 183.0899 $C_9H_{13}NO_3$ | 183.0895 |
| 9 | 197(18) | $M^+$ 197.1048 $C_{10}H_{15}NO_3$ | 197.1052 |
| 10 | 183(17) | $M^+$ 183.0895 $C_9H_{13}NO_3$ | 183.0895 |
| 11 | 197(37) | $M^+$ 197.1051 $C_{10}H_{15}NO_3$ | 197.1052 |
| 12 | 125(100) | $M^+$ 125.0477 $C_6H_7NO_2$ | 125.0477 |
| 13 | 165(100) | $M^+$ 165.0790 $C_9H_{11}NO_2$ | 165.0785 |
| 14 | 183(100) | $M^+$ 183.0895 $C_9H_{13}NO_3$ | 183.0899 |
| 15 | 199(100) | $M^+$ 199.0851 $C_9H_{13}NO_4$ | 199.0845 |
| 16 | 179(57) | $M^+$ 179.0945 $C_{10}H_{13}NO_2$ | 179.0946 |
| 17 | 241(29) | $M^+$ 241.1314 $C_{12}H_{19}NO_4$ | 241.1319 |
| 18 | 224(2) | $M^+$ 224.1531 $C_{12}H_{20}N_2O_2$ | 224.1525 |
| 19 | 211(85) | M+ 211.1211 $C_{11}H_{17}NO_3$ | 211.1208 |
| 20 | 225(26) | M+ 225.1364 $C_{12}H_{19}NO_3$ | 225.1365 |

TABLE 4

| Compound of Example No | Extinction Coefficient Chelator | $M^{-1}cm^{-1}$ Iron Complex | PKa(1) | Pka(2) | K par Chelator | K par Iron Complex |
|---|---|---|---|---|---|---|
| 4 | 13800 (282nm) | 4910 (470nm) | 3.7 | 9.8 | 0.4 | 0.02 |
| 5 | 14100 (282nm) | 5000 (470nm) | 3.7 | 10.1 | 1.4 | 0.28 |
| 6 | 14500 (283nm) | 5050 470nm) | 3.4 | 10.1 | 4.2 | 9.68 |
| 1 | 13300 (280nm) | 5040 (460nm) | 3.3 | 9.7 | 0.19 | 0.24 |
| 2 | 13800 (282nm) | 5140 (460nm) | 3.6 | 10.3 | 0.37 | 0.52 |
| 3 | 14200 (280nm) | 5170 (460nm) | 3.7 | 10.2 | 3.16 | 4.12 |

TABLE 5

| Example No. | | 2-Alkyl-3-hydroxypyr-4-one 30.00g Yield[N*] %Yield** | 80.00g |
|---|---|---|---|
| 4 | 2-Ethyl-1-Methyl | 4.64g[3] 14.2% | 28.98g[5] 33.1% |
| 5 | 1,2-Diethyl | 1.93g[3] 5.4% | 20.70g[5] 21.7% |
| 6 | 2-Ethyl-1-propyl | 2.00g[2] 5.2% | 13.47g[3] 13.0% |
| 1 | 1,2 Dimethyl | 19.27g[2] 58.2% | 54.92g[2] 62.2% |
| 2 | 1-Ethyl-2-methyl | 16.56g[3] 45.5% | 33.78g[3+] 37.9% |
| 3 | 2-Methyl-1-propyl | 10.56g[2] 26.6% | 21.72g[2=] 32.8% |

* N = Number of recrystallisations
\+ From 70.00g maltol
= From 50.00g maltol
** No attempt has been made to optimise yields

EXAMPLE 20

Three novel iron chelators namely the 1-methyl, 1-ethyl and 1-propyl -2-ethyl-3-hydroxypyrid-4-ones were prepared in high yields from ethyl maltol and the related alkylamine in a one step reaction. These chelators formed 3 chelator: 1 iron stable, coloured neutral complexes at physiological pH and mobilise iron from transferrin, ferritin and haemosiderin. The rate of iron mobilisation from these proteins was of the order transferrin > haemosiderin > ferritin. The cheap synthesis and strong iron binding properties of the 1-alkyl-2-ethyl-3-hydroxypyrid-4-ones at physiological pH requires the need for further investigation and development of these compounds and their homologues, for the treatment of iron overload and other diseases or iron imbalance and toxicity.

The basic properties of an ideal chelator intended for clinical use are inexpensive synthesis, oral activity and low toxicity. The design of such chelators would save the lives of many iron loaded thalassaemia patients who would otherwise die untreated because of the high cost of desferrioxamine or due to non compliance with the 8-10h long daily infusion treatment with this drug. [1-2].

Iron has also been implicated in the pathogenesis of several other diseases, where its catalytic formation of toxic oxygen activated products such as superoxide, hydrogen peroxide and hydroxyl radical may result in tissue damage [3-4]. The design of site-specific chelators may be needed to deal with such diseases [5]. Other uses of chelators include the removal of other metals such as Pu and Al [6,7] which have similar metabolic pathways to iron and design of lipophilic iron complexes for the treatment of iron deficiency anaemia.

The design of a new potent group of iron chelators, namely the α-ketohydroxypyridines by Kontoghiorghes [8-11] and the identification of the 1-alkyl-2-methyl-3-hydroxypyrid-4-ones as the most promising sub group in the mobilisation of iron in vivo [12-14] led us recently to undertake the first clinical trials in the treatment of iron overload using the oral chelator 1,2-dimethyl-3-hydroxypyrid-4-one (LI) [15] and design of new 1,2-dialkyl-3-hydroxypyrid-4-ones.

In this Example we report the synthesis and characterisation of a novel group of iron chelators namely the 1-alkyl-2-ethyl-3-hydroxypyrid-4-ones. In addition we have examined the iron complex formation and the iron mobilisation properties of these chelators from transferrin, ferritin and haemosiderin in vitro.

Iron Binding Studies

pH titration curves of the chelator-iron complexes were obtained using a mixture of the chelators (25ml; 2mM in 0.1 M NaCl) and freshly prepared iron (III) chloride solution (25ml, 0.5mM in 0.1 M NaCl), and titrating with HCl (10M) and NaOH (5M)(Fig.1). Job plots of the chelators (0-5ml, 2mM in 0.1M NaCl) and iron (0-5ml, 2mM in 0.1M NaCl) at selected molar ratios were carried out at pH 7.3 using 4 x PBS (Phosphate buffered saline, 5ml) as previously described [17]. These were compared to theoretical plots of 1:1, 2:1 and 3:1 chelator:iron complexes (Fig.2).

The n-octanol/water (PBS) partition coefficients (K par) of the chelators and their iron complexes were

determined using a previously determined method [8].

Iron Mobilisation from Transferrin, Ferritin and Haemosiderin

Human apotransferrin (Sigma) was radioactively labelled with $^{59}Fe$ and saturated with iron as previously described [18]. Samples (1.5ml) of the $^{59}Fe$ transferrin (0.54mg protein, 0.75 μg iron, 0.07 μCi $^{59}Fe$) were enclosed in a dialysis bag and dialysed against a chelator solution (10ml, 4mM) by continuous stirring at 37° C. Samples (1ml) of the dialysate were removed at different time intervals, the $^{59}Fe$ activity measured and then returned back into the incubation mixture. Ferritin solution and haemosiderin solid were isloated from a spleen of a thalassemia patient [19] and samples containing the same amount of iron (0.25mg, 1.5ml) were dialysed at 37° C against a chelator solution (4mM, 10ml) over a 12 day period. The amount of iron immobilised was estimated spectrophotometrically by measuring the visible absorbance of the dialysate and by using the extinction coefficient of the chelator iron complex (Fig 3, Table 6). All the protein incubations with the chelators were carried out in the 2xPBS pH 7.3.

Results

Coloured complexes were formed when mixing the 1-alkyl-2-ethyl-3-hydroxypyrid-4-ones with iron which are characterised by similar pH titration curves (Fig.1.) containing a plateau region above annd below the physiological pH. The Job plots at pH 7.4 revealed the formation of a tris chelator to one iron complex which predominates over this plateau region (Fig.2). Lower than 3:1 chelator to iron ratio complexes are expected to be formed at acidic pH'S similar to the 1-alkyl-3-hydroxy-2-methylpyrid-4-ones and 4-substituted-1-hydroxy-pyrid-2-ones [10,17]. The pKas of all three 1-alkyl-2-ethyl-3-hydroxy pyrid-4-ones were very similar (Table 4). The pKa of the hydroxyl group was sufficiently high (>9.0) for the formation of neutral molecules at pH. In contrast, the n-octanol/water (PBS) partition coefficients of these chelators were found to increase with increasing the size of the 1-alkyl substitution (Table 4).

Iron mobilisation by the 1-alkyl-2-ethyl-3-hydroxypyrid-4-ones from human transferrin progressively increased with time and was higher than 90% after 8h of incubation (Table 6). In contrast haemosiderin and ferritin iron release by these chelators was much slower, and a smaller percentage of iron was released following several days of incubation (Fig 3.). Haemosiderin iron release was by comparison faster than ferritin as previously shown by other chelators [19].

Discussion

It was initially decided to proceed with the synthesis of new 1-alkyl substituted 2-ethyl-3-hydroxypyrid-4-ones because previously, 1-alkyl substituted 2-methyl-3-hydroxypyrid-4-ones were identified as more effective in vivo than other α-ketohydroxypyridines [14]. The quantitative synthesis of all three 1-alkyl-2-ethyl-3-hydroxyopyrid-4-ones from ethyl maltol in a one step reaction, which is similar to that of 1-alkyl-3-hydroxy-2-methylpyrid-4-ones from maltol, fulfills one of the basic criteria of iron chelators intended for clinical use, that is to be cheap as explained in the introduction.

The 1-alkyl-2-ethyl-3-hydroxypyrid-4-ones and their iron complexes were found to be highly stable in air and in solutions of variable pHs (Fig.1, Table 4). Using a theoretical model the stoichiometry of the complex with iron was easily identified as neutral, with a 3:1 chelator:iron ratio at a wide range of pH (6-11), including the physiological pH (Fig.2). This property ensures the maintenance of a buffer capacity by the neutral complex over a wide range of pH usually found in in vivo media. At physiological pH the neutral charge of the chelators and their iron complexes will also facilitate the permeability towards cell membranes, which, depending upon the K par of the molecules, could either result in an increase in iron donation (by lipophilic chelators) or iron witholding (by hydrophilic chelators) in cells [16,20]. In addition, 2-ethyl-3-hydroxy-1-propylpyrid-4-one which is the most lipophilic of the three new derivatives is expected to be highly toxic in vivo as previously shown with the most lipophilic of the 1-alkyl-3-hydroxy-2-methylpyrid-4-ones, namely the 3-hydroxy-2-methyl-1-propylpyrid-4-one [16].

Substantial iron mobilisation from transferrin, ferritin and haemosiderin took place following their incubation with 1-alkyl-2-ethyl-3-hydroxypyrid-4-ones. The ability of these chelators to mobilise transferrin iron makes them one of the most effective group of iron chelators known. Transferrin iron was more rapidly mobilised in comparison to that found as a polynuclear form in ferritin and haemosiderin. While almost all the iron was released from transferrin within 8 h (Table 6), iron mobilisation from haemosiderin and ferritin was slow and only a fraction was mobilised following days of incubation (Fig.3). Haemosiderin iron mobilisation was faster than ferritin which is in agreement with our previous observations [19]. It is expected therefore that in vivo iron would be available for chelation from all three iron pools. The comparative studies of iron mobilisation from transferrin, ferritin and haemosiderin, and also the iron binding affinities shown in the Job plot studies indicate that the three 1-alkyl-2-ethyl-3-hydroxypyrid-4-ones have similar kinetic and thermodynamic iron binding constants despite their differences in n-octanol/water partition. However, differences in K par may result in differences in in vivo activity as previously suggested [14,16].

TABLE 6

IRON MOBILISATION FROM TRANSFERRIN, FERRITIN AND HAEMOSIDERIN

| 3-Hydroxy pyrid-4-one ($4x10^{-3}M$) | Percentage Iron Removal: Transferrin $^{59}Fe$ 1h | 2h | 8h | Haemosiderin 24h | 12d | Ferritin 24h | 12d |
|---|---|---|---|---|---|---|---|
| 1,2-Dimethyl (Example 1) | 37.3 | 69.9 | 99.5 | | | | |
| 1-Methyl-2-ethyl (Example 4) | 36.0 | 56.6 | 100.0 | 26.7<br>25.2* | 58.8<br>62.7* | 9.4<br>9.9* | 27.0<br>29.0* |
| 1,2-Diethyl (Example 5) | 24.9 | 51.8 | 96.6 | 23.1<br>22.7* | 51.0<br>62.7* | 8.1<br>8.5* | 30.6<br>29.8* |
| 1-Propyl-2-ethyl (Example 6) | 23.2 | 50.3 | 97.6 | 27.8<br>- | 74.5<br>- | 8.5<br>8.3* | 33.9<br>27.0* |

* Duplicate samples.

Haemosiderin (0.25mg iron, 16.5% w/w iron, 15ml) ferritin (0.25mg iron, 10.7% w/w iron, 1.5ml) and transferrin (0.75ug iron, 0.54mg protein, 0.07 uCi $^{59}Fe$, 1.5ml) in dialysis bags were dialysed against the chelators ($4x10^{-3}M$, 10ml) at 37°C, pH 7.3 in 2 x PBS by continuous stirring. Iron mobilisation was estimated from samples of the dialysates taken at different time intervals as explained in the Materials and Methods section.

REFERENCES FOR EXAMPLE 20

1. Bulletin of the World Health Organisation 61 (i),63 (1983)
2. E.G. Brown
Progr. Clin. Biol. Reseach 127,33 (1983).
3. Editorial.
Lancet i:143 (1985).
4. G.J. Kontoghiorghes, M.J. Jackson, J. Lunec
Free Rad. Res. Commun. 2,115 (1986).
5. G.J. Kontoghiorghes in C Rice-Evans (ed).
Free Radicals, Oxidant Stress and Drug Action. 227 (1987)
6. D.M. Taylor, G.J. Kontoghiorghes
Inorg. Chim. Acta. 125, L35 (1986).
7. J.E. Coburn, K.C. Norris.
Seminars in Nephrology. 6,42 (1986).
8. G.J. Kontoghiorghes. Ph.D Thesis, Essec University British Library Microfilm D66194.86, 1982.
9. G.J. Kontoghiorghes. Lancet i, 817 (1985).
10. G.J. Kontoghiorghes. Inorg, Chim, Acta 135, 145 (1987)
11. G.J. Kontoghiorghes. Biochem. J. 233, 299 (1986).
12. G.J. Kontoghiorghes. Scand. J. Haematol 37, 63 (1986).
13. G.J. Kontoghiorghes. A.V. Hoffbrand,
Brit. J. Haematol 62, 607 (1986)
14. G.J. Kontoghiorghes. Molec. Pharmacol. 30, 670 (1986).
15. G.J. Kontoghiorghes, M. Aldouri, L. Sheppard, A.V. Hoffbrand Lancet 1,1294 (1987).
16. G.J. Kontoghiorghes, L. Sheppard, Inorg, Chim, Acta 136, L11 (1987).
17. G.J. Kontoghiorghes, L. Sheppard, S. Chambers.
Arxenimitrel-Forsch/Drug Res. 37 1099 (1987).
18. G.J. Kontoghiorghes, L. Sheppard, A.V. Hoffbrand,
Charalambous J, Tikerpae J, Pippard M.J. J. Clin. Path. 40, 404 (1987).
19. G.J. Kontoghiorghes, S. Chambers, A.V. Hoffbrand. Biochem. J. 241, 87 (1987).
20. G.J. Kontoghiorghes. Inorg. Chim. Acta 151,101 (1988).

EXAMPLE 21

Partition Coefficients of the Chelators and Red Blood Cell Permeability Studies of the Chelator Iron Complexes

The partition coefficients of the chelators and their iron complexes were determined using a mixture of n-octanol and phosphate buffer saline at pH 7.3 as previously described [9,10,11]. The effect of the chelators on the incorporation of iron containing traces of $^{59}Fe$ into red blood cells was studied as previously described [10,11] by incubating the chelator iron complexes with packed red blood cells at 37°C, withdrawing aliquots, centrifuging and measuring the incorporation of $^{59}Fe$ into the red blood cells.

Studies in Animals

The three 1-alkyl-3-hydroxy-2-methylpyrid-4-ones dissolved in phosphate buffer saline were administered intraperitoneally to groups of three male Sprague Dawley rats (60-90g) at different doses until a near maximum non-lethal dose was established.

Iron mobilisation from iron loaded $^{59}Fe$ transferrin labelled mice by the 1-alkyl-2-ethyl-3-hydroxypyrid-4-ones was studied as previously described [12].

Results and Discussion

The partition coefficients of the chelators and their iron complexes were shown to increase as the chain length of the N-alkyl substituents was increasing (Table 7).

The lower lethal dose observed in rats treated with 3-hydroxy-2-methyl-1-propylpyrid-4-one in comparison to those treated with the other two derivatives may be related to the higher Kpar of the former (Table 7). Although doses of up to 200 mg/kg of the 1-methyl and 1-ethyl-2-methyl-3-hydroxypyrid-4-ones were shown to be effective in increasing iron excretion in loaded mice and rabbits [3] and also in non iron loaded mice [4], further studies are required to establish therapeutic and lethal doses in rats and other animals and in man [13].

The three 1-alkyl-2-ethyl-3-hydroxypyrid-4-one chelators were found to be effective in the mobilisation of $^{59}Fe$ from mice when administered either ip or ig. The level of $^{59}Fe$ excretion caused by the 1-alkyl-2-ethyl-3-hydroxypyrid-4-ones was similar to that of desferrioxamine administered ip. The $^{59}Fe$ excretion caused by all three chelators were slightly higher with the ip than the ig route of administration (Table 8). This small difference indicates that sufficient amounts of the 1-alkyl-2-ethyl-3-hydroxypyrid-4-ones are absorbed from the gastrointestinal tract following an ig administration which cause comparative levels of $^{59}Fe$ excretion to those observed via the ip route.

2-Ethyl-3-hydroxy-1-propylpyrid-4-one caused death to two mice before the completion of the treatment. The lethal toxicity of the propyl derivative following its administration at the same dose as the methyl and ethyl derivatives may be related to the higher lipophilicity of the former (Table 8) as previously shown in the 1-alkyl-2-methyl-3-hydroxypyrid-4-ones.

The red blood cell membrane permeability of iron in the presence of the chelators seems also to be related to the partition coefficient of the chelator iron complexes (Table 7) which is in agreement with previous observations [9]. Lipophilic iron chelator complexes such as that of 3-hydroxy-2-methyl-1-propylpyrid-4-one which could easily diffuse through membranes may have a use in the treatment of iron deficiency anaemia by increasing iron absorption.

The low cost of synthesis, apparent low toxicity and high efficacy in increasing iron excretion via the ig route in mice by the 1-methyl, 1-ethyl- and 1-propyl 2-ethyl-3-hydroxypyrid-4-ones increases the prospects for the use of these two chelators and other related non toxic derivatives in the treatment of transfusional iron overload and other diseases of iron imbalance and toxicity.

TABLE 7

Properties of the Chelators and their Iron Complexes

| Derivatives of 3-hydroxy-2-methylpyrid-4-ones | Lethal Dose (range)[b] (mg/kg) | Kpar[a] Chelator | Kpar[a] iron Complex | $^{59}Fe$ incorporation (%) | |
|---|---|---|---|---|---|
| | | | | 20 min | 1h |
| 1-Methyl | 600-700 | 0.19 | 0.24 | 5.0 | 5.0 |
| 1-Ethyl | 400-500 | 0.47 | 0.52 | 5.0 | 10 |
| 1-Propyl | 150-200 | 3.16 | 4.12 | 85 | 86 |

[a]Kpar is the n-octanol/phosphate buffer saline (PH 7.3) partition coefficient of the chelator.
[b]Range of doses, the higher being lethal and the lower a non-lethal dose.
[c]The percentage incorporation of traces of $^{59}Fe$ mixed with iron ($2.5 \times 10^{-5}$M) into red blood cells in the presence of the chelators ($18 \times 10^{-5}$M) was estimated from the total $^{59}Fe$ count of red blood cells plus supernatant/oil (100% $^{59}Fe$ $1.4 \times 10^{3}$ cpm).

TABLE 8

Iron Mobilisation from Mice by the 1-Alkyl-2-ethyl-3-hydroxy-pyrid-4-ones

| Compound | Route and Number of administrations[a] | %$^{59}$Fe excretion[b] X ± SE |
|---|---|---|
| 2-ethyl-1-methyl | 15 ip | 415 ± 42 |
| 3-hydroxy-pyrid-4-one | 15 ig | 362 ± 53 |
| 1,2-diethyl-3- | 15 ip | 371 ± 46 |
| hydroxypyrid-4-one | 15 ig | 309 ± 22 |
| 2-ethyl-3-hydroxy | 11 ip[c] | 334 ± 64 |
| 1-propylpyrid-4-one | 11 ig[c] | 163 ± 25 |
| Desferrioxamine | 7 ip | 381 ± 49 |

a. The dose was 10mg and the weights of mice 31-35g
b. The mean %$^{59}$Fe excretion of 3 days before the chelator administration was taken as 100% and all daily $^{59}$Fe excretions were estimated against this mean. See reference 12.
c. Two mice died in this group.

REFERENCES TO EXAMPLE 21


1. Bulletin of the World Health Organisation, 63 (i), 63 (1983).
2. E.G. Brown, Prog. Clin. Biol. Res., 127, 33 (1985).
3. G.J. Kontoghiorghes and A.V. Hoffbrand, Br. J. Haematol, 62, 607
4. G.J. Kontoghiorghes, Lancet, I, 817 (1985)
5. G.J. Kontoghiorghes. Bioch. Biophys, Acta 869, 141 (1986).
6. G.J. Kontoghiorghes, Biochem. J. 233, 199 (1986).
7. T. Severin and A. Loidl, Z. Lebensm, Unters-Forsch., 161, 119, (1976).
8. M. Yasue, N. Kawamura and J. Sakakibara, Yakugaku Zasshi, 90, 122 (1970)
9. G.J. Kontoghiorghes, Ph.D. Thesis Essex University British Library Microfilm D66194/86 1982).
10. G.J. Kontoghiorghes, A. Piga and A.V. Hoffbrand, FEBS Lett., 204, 208 (1986).
11. G.J. Kontoghiorghes, Inorg. Chim. Acta. 151,101 (1988).
12. G.J. Kontoghiorghes, Molec. Pharmacol. 30, 670 (1986).
13. G.J. Kontoghiorghes, M. Aldouri, L. Sheppard, A.V. Hoffbrand, Lancet, i; 1294 (1987).


**Claims**

1. A process for producing a compound selected from substituted-3-hydroxypyrid-4-ones and derivatives thereof and salts thereof, said process comprising reacting a substituted-3-hydroxypyr-4-one with a reagent selected from ammonia, non-sterically hindered primary amines and acids addition salts thereof.

2. A process according to claim 1 comprising reacting a 2-substituted-3-hydroxypyr-4-one with said reagent.

3. A process according to claim 2 comprising reacting 2-alkyl-3-hydroxypyr-4-one with said reagent.

4. A process according to claim 3 comprising reacting 2-ethyl- or 2-methyl-3-hydroxypyr-4-one with said reagent.

5. A process according to claim 1 wherein said reagent is ammonia or a hydrohalic acid salt thereof.

6. A process according to claim 1 wherein said reagent is a primary alkylamine or a hydrohalic acid

addition salt thereof.

7. A process according to claim 6 wherein said primary alkylamine is selected methylamine, ethylamine, N-propylamine and hydrohalic acid addition salts thereof.

8. A process according to claim 6 wherein said primary alkylamine bears at least one substituent on the alkyl group, each said substituent being selected from hydroxy, carboxy, alkenyl, alkoxy, acyl, acyloxy, amino, mono-alkylamino and di-alkylamino.

9. A process according to claim 1 wherein said reagent comprises more than non-sterically hindered primary amino group.

10. A process according to claim 1 conducted in the presence of a solvent.

11. A process according to claim 10 wherein said solvent is selected from water, lower alkanols and aqueous lower alkanols.

12. A process according to claim 10 conducted at the reflux temperature of the reaction mixture.

13. A process according to claim 1 comprising the addition steps of isolation and purification of the substituted-3-hydroxypyrid-4-one or derivative or salt thereof.

14. A compound selected from 2-ethyl-3-hydroxypyrid-4-one, derivatives thereof and acid addition salts of 2-ethyl-3-hydroxypyrid-4-one and said derivatives.

15. A compound selected from N-alkenyl-2-alkyl-3-hydroxypyrid-4-ones and acid addition salts thereof.

16. A compound according to claim 15 selected from N-allyl-2-alkyl-3-hydroxypyrid-4-ones and salts thereof.

17. A compound according to claim 15 selected from N-alkenyl-2-ethyl-3-hydroxypyrid-4-ones and acid addition salts thereof.

18. A compound according to claim 14 and selected from
1,2-diethyl-3-hydroxy-pyrid-4-one,
2-ethyl-3-hydroxy-1-propylpyrid-4-one,
2-ethyl-3-hydroxypyid-4-one,
3-hydroxy-1-(2-methoxyethyl)-2-methylpyrid-4-one,
2-ethyl-3-hydroxy-1-(2-hydroxypropyl)pyrid-4-one,
2-ethyl-3-hydroxy-1-(2-hydroxyethyl)-pyrid-4-one,
2-ethyl-3-hydroxy-1-(2-methoxyethyl)pyrid-4-one,
3-hydroxy-2-methylpyrid-4-one,
1-allyl-3-hydroxy-2-methylpyrid-4-one,
3-hydroxy-1-(2-hydroxypropyl)-2-methylpyrid-4-one,
1-(2,3-dihydroxypropyl)-3-hydroxy-2-methylpyrid-4-one,
1-allyl-2-ethyl-3-hydroxypyrid-4-one,
1-(2,2-diethoxyethyl)-3-hydroxy-2-methylpyrid-4-one,
1-(2-diethylaminoethyl)-3-hydroxy-2-methylpyrid-4-one,
1-(3-ethoxypropyl)-3-hydroxy-2-methylpyrid-4-one,
1-(3-ethoxypropyl)-2-ethyl-3-hydroxypyrid-4-one and
acid addition salts thereof.

19. A pharmaceutical formulation comprising a pharmaceutically effective non-toxic amount of a compound according to claim 14 together with a pharmaceutically acceptable carrier or diluent therefor.

20. A pharmaceutical formulation comprising a pharmaceutically effective non-toxic amount of a compound according to claim 15 together with a pharmaceutically acceptable carrier or diluent therefor.

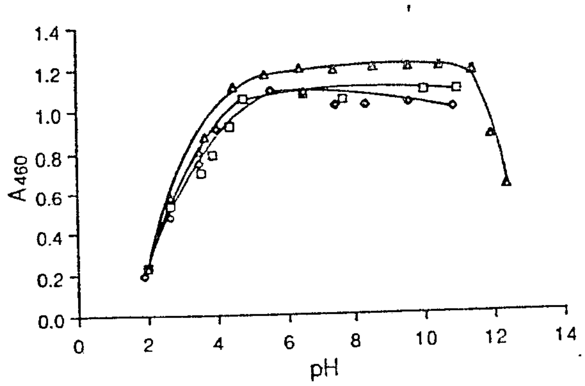
1.4
1.2
1.0
0.8
0.6
0.4
0.2
0.0
A460
0
2
4
6
8
10
12
14
pH

FIG. 1.

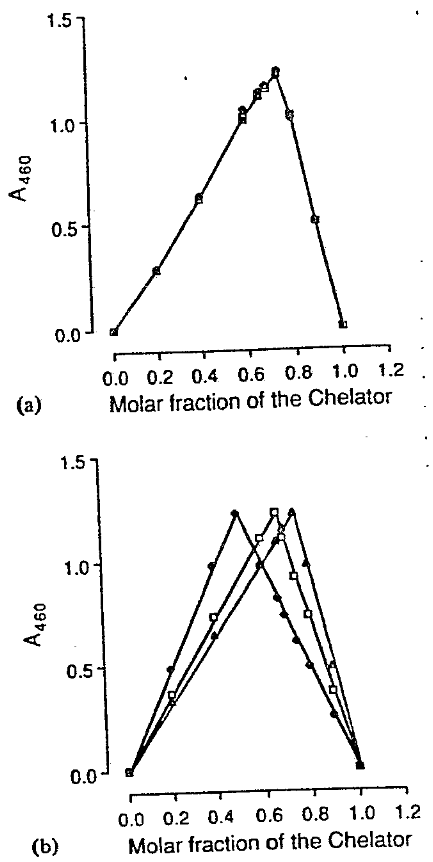
1.5
1.0
0.5
0.0
A460
0.0 0.2 0.4 0.6 0.8 1.0 1.2
(a)
Molar fraction of the Chelator
1.5
1.0
0.5
0.0
A460
0.0 0.2 0.4 0.6 0.8 1.0 1.2
(b)
Molar fraction of the Chelator

FIG. 2.

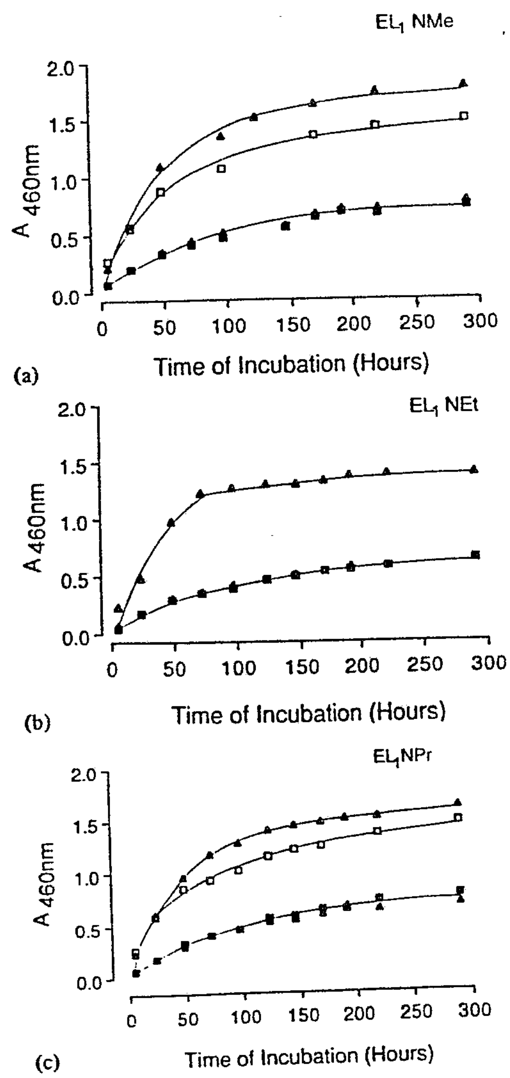
EL1 NMe
2.0
1.5
1.0
0.5
0.0
A 460nm
0
50
100
150
200
250
300
Time of Incubation (Hours)
(a)
EL1 NEt
A 460nm
Time of Incubation (Hours)
(b)
EL1NPr
A 460nm
Time of Incubation (Hours)
(c)

FIG. 3.

European Patent Office

# EUROPEAN SEARCH REPORT

Application Number

EP 89 30 3228

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 74, 1971, page 339, abstract no. 23102b, Columbus, Ohio, US; M. YASUE et al.: "Syntheses of N-substituted-3-glucosyloxy-2-methyl-4-pyridones and their aglycones", & YAKUGAKU ZASSHI 1970, 90(10), 1222-5<br>* Abstract *<br>--- | 18 | C 07 D 213/69<br>A 61 K 31/44 |
| X | CHEMICAL ABSTRACTS, vol. 102, 1985, page 695, abstract no, 113315z, Columbus, Ohio, US; & JP-A-59 161 359 (TOKUYAMA SODA CO., LTD) 12-09-1984<br>* Abstract *<br>--- | 18 | |
| A | EP-A-0 120 669 (NATIONAL RESEARCH DEVELOPMENT CORP.)<br>* Whole document *<br>--- | 1,14,18,19 | |
| A | EP-A-0 093 498 (NATIONAL RESEARCH DEVELOPMENT CORP.)<br>* Claims *<br>--- | 1,14,18,19 | TECHNICAL FIELDS SEARCHED (Int. Cl.4) |
| P,X | CHEMICAL ABSTRACTS, vol. 110, 1989, abstract no. 18109s, Columbus, Ohio, US; J.B. PORTER et al.: "Iron mobilization from hepatocyte monolayer cultures by chelators: the importance of membrane permeability and the iron-binding constant", & BLOOD 1988, 72(5), 1497-503<br>* Abstract *<br>--- -/- | 18,19 | C 07 D 213/00 |

The present search report has been drawn up for all claims

| Place of search | Date of completion of the search | Examiner |
|---|---|---|
| THE HAGUE | 07-07-1989 | VAN BIJLEN H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

EP 89 30 3228

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,X | CHEMICAL ABSTRACTS, vol. 110, 1989, pages 326-327, abstract no. 91133z, Columbus, Ohio, US; R.C. HIDER et al.: "The inhibition of tyrosinase by pyridinones", & BIOCHEM. J. 1989, 257(1), 289-90<br>* Abstract *<br>----- | 18 | |

TECHNICAL FIELDS SEARCHED (Int. Cl.4)

The present search report has been drawn up for all claims

| Place of search | Date of completion of the search | Examiner |
|---|---|---|
| THE HAGUE | 07-07-1989 | VAN BIJLEN H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document